# EUROPEAN PATENT SPECIFICATION

(11) **EP 4 041 158 B1**
(45) Date of publication and mention of the grant of the patent: **26.11.2025**
(21) Application number: 20792742.7
(22) Date of filing: 06.10.2020
(51) Int. Cl.: A61F 13/00, A61F 13/02

(54) **A WOUND DRESSING**
WUNDVERBAND
PANSEMENT

(30) Priority: 07.10.2019 US 201962911760 P
(43) Date of publication of application: 17.08.2022
(73) Proprietor: Solventum Intellectual Properties Company, Maplewood, MN 55144 (US)
(72) Inventor: HOLM, David R., Saint Paul, Minnesota 55133-3427 (US); JACOBSON, Richard L., Saint Paul, Minnesota 55133-3427 (US)
(74) Representative: Vossius & Partner Patentanwälte Rechtsanwälte mbB
(86) International application number: PCT/IB2020/059383
(87) International publication number: WO 2021/070058

(56) References cited:
- EP-A1- 3 089 719
- US-A- 5 147 338
- US-A1- 2014 188 090
- US-A1- 2019 231 604

## Description

### BACKGROUND

Many different types of skin traumas benefit from preventing infection and promoting healing. Such traumas are often cared for with dressings that protect the area from microorganisms, dirt and debris, and further trauma. In some instances, dressings may allow for delivery and/or localizing of therapeutics. However, not all skin traumas are easy to effectively dress or cover. For example, long injuries such as surgical incisions can be difficult to cover because they are not always linear, or they often require the use of multiple dressings partly because dressings of greater size are not easily handled or available. The use of multiple dressings requires cutting and customizing of the dressings which is time consuming and can result in difficult application of the dressing which can result in poor coverage and scaling of the injury from contaminants. Likewise, nonlinear traumas can require the use of multiple dressings because the dressing is not able to flex or bend adequately during application. In addition, traumas that traverse nonplanar surfaces, e.g., long and/or nonlinear traumas over bendable surfaces, suffer from the lack of adequate dressing conformability coupled with adequate support to handling during application to the nonplanar surface of the body.

Despite the advances in wound dressing systems, there remains a need for wound dressings that are conformable, resilient, and easy to use for long and relatively narrow types of skin traumas. EP 3 089 719 A1 discloses a wound dressing system which includes a wound dressing and a dressing support layer. The wound dressing comprises a backing with two major surfaces, first and second lateral edges, an adhesive disposed on one major surface and the dressing support layer removably mounted on the other major surface. The dressing support layer comprises juxtaposed first and second sections that define a gap between them. Each section comprises a medial portion and at least four spokes extending therefrom toward a lateral edge. The at least four spokes comprise two outer spokes and at least two spaced-apart inner spokes. At least two of the at least four spokes of each section extend beyond the lateral edge to form peripheral support layer tabs.

### SUMMARY

The invention is defined by the features of claim 1. Preferred embodiments are defined by the features of the dependent claims.

### BRIEF DESCRIPTION OF DRAWINGS

**FIG. 1A** is a schematic top plan view of a wound dressing of the present disclosure
**FIG. 1B** is a schematic cross-sectional view of the wound dressing in, for example, **FIG. 1A****.**
**FIG. 1C** is a schematic cross-sectional view of the wound dressing in, for example, **FIG. 1A****.**
**FIG. 2** is a schematic top plan view of a wound dressing of the present disclosure.
**FIG. 3** is a schematic top plan view of one embodiment of a wound dressing.
**FIG. 4** is a schematic top plan view of one embodiment of a wound dressing.
**FIG. 5** is a schematic top plan view of one embodiment of a wound dressing.
**FIG. 6** is a schematic top plan view of one embodiment of a wound dressing.
**FIG. 7** is a schematic top plan view of one embodiment of a wound dressing.
**FIG. 8** is a schematic top plan view of one embodiment of a wound dressing.
**FIG. 9** is a flow chart of a method of manufacturing the wound dressings of the present disclosure.
**FIG. 10** is a flow chart of a method of using the wound dressings of the present disclosure.

### DETAILED DESCRIPTION

The disclosed wound dressing(s) include an elongated composite and a delivery system with at least three disconnected support networks that allow for enhanced handling of the dressing and flexibility in placement over wounds, especially long and narrow wounds or non-linear wounds.

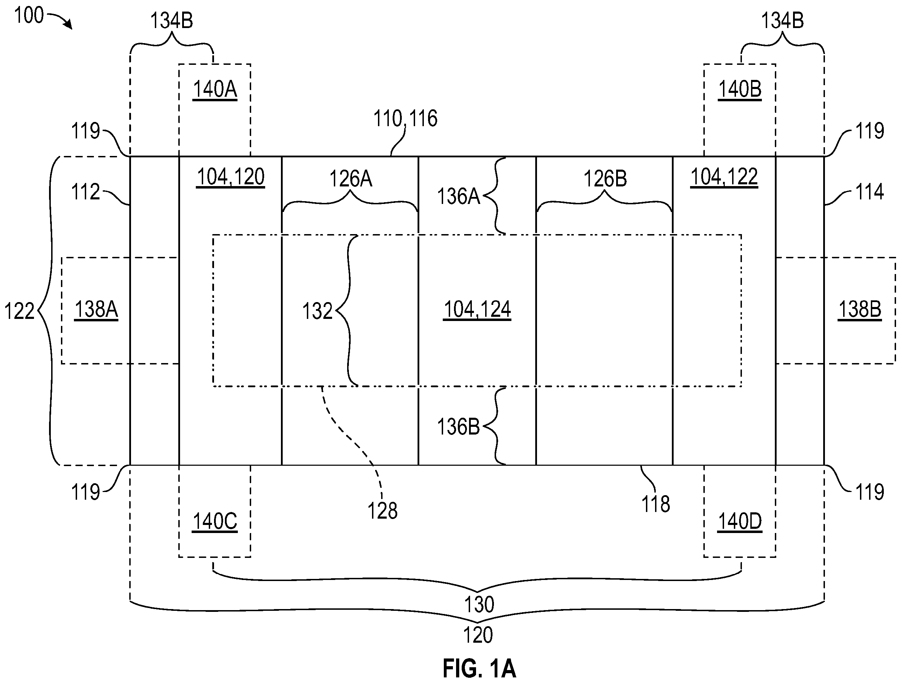

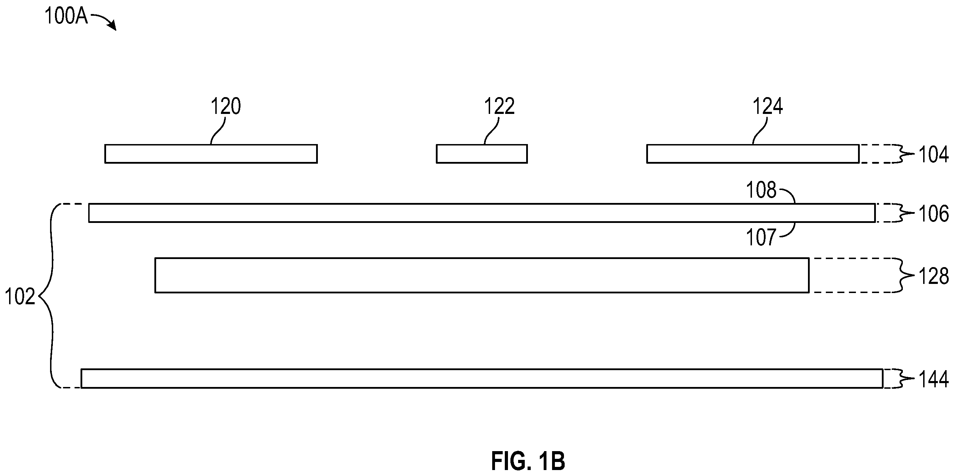

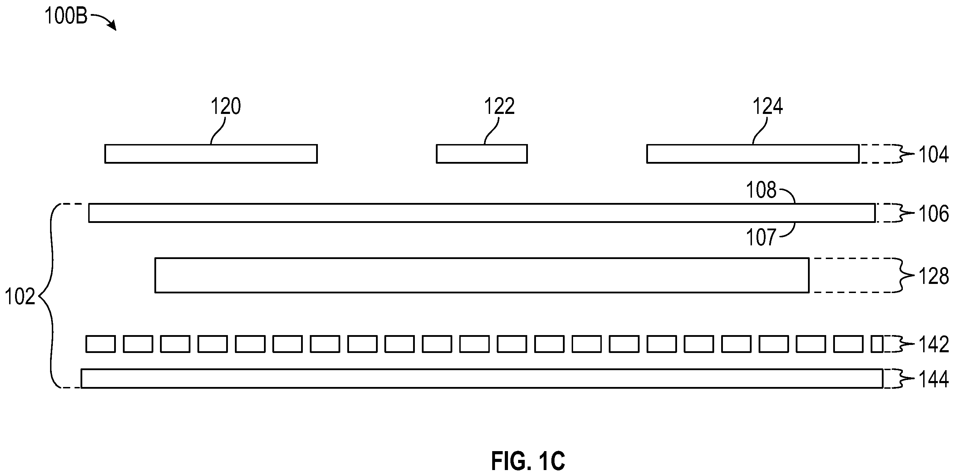

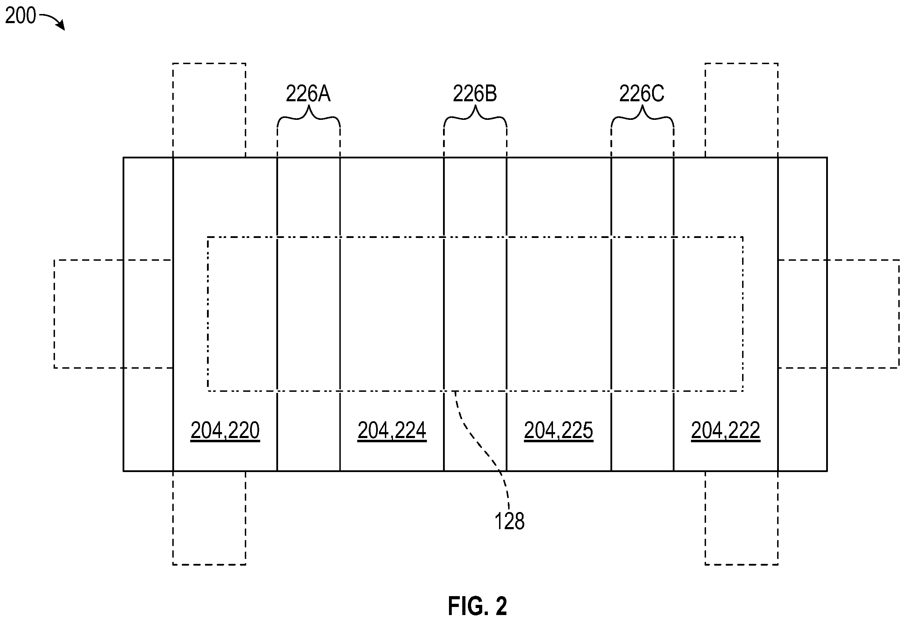

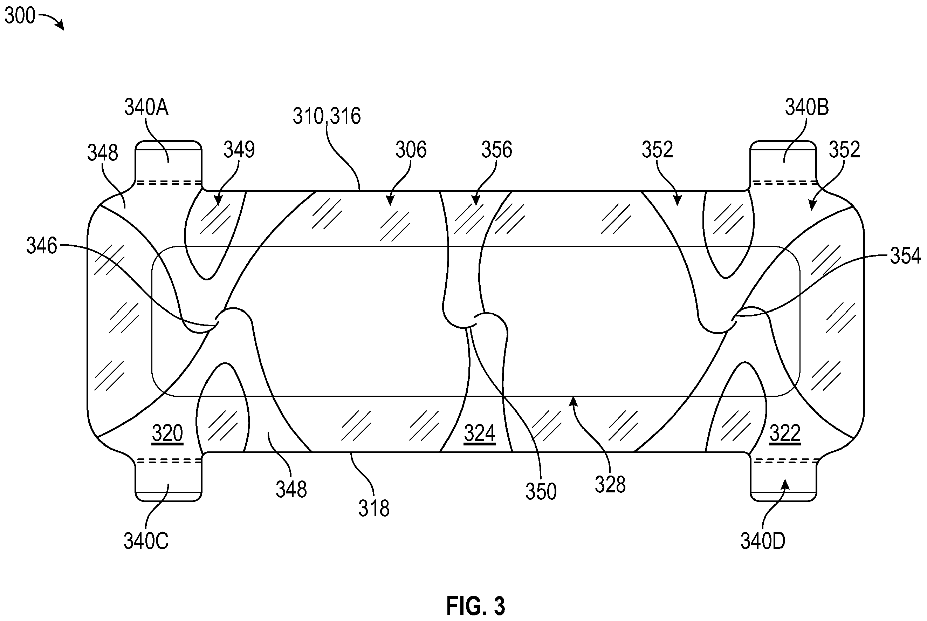

The following figure descriptions provide numerical identifiers for the features described. The second and third significant figure in each numerical identifier is conserved with respect to figure feature. For example, a first support network in figure 1 is 120, in figure 2 is 220, in figure 3 is 320, and so forth. It is to be understood that not all features are identified numerically in each figure in order to promote visual clarity.

**FIG. 1A** and **FIG. 2** generally relate to wound dressings of the present disclosure. **FIGs. 3-8** describe example wound dressings of the present disclosure. It is to be understood that any features of one embodiment, unless indicated otherwise, may be included or combined with features of other embodiments described herein.

**FIG. 1** illustrates a wound dressing **100.** Wound dressing **100** includes an elongated composite (not shown, see **102** in FIG. 1A and 1B) and a delivery system **104.** Elongated composite **(102)** includes a flexible substrate (not shown, see **106** in FIG. 1A and 1B) and a perimeter **110.** The flexible substrate includes a first surface (not shown, see **107** in FIG. 1A and 1B) and a second surface (not shown, see **108** in FIG. 1A and 1B) opposite the first surface. Perimeter **110** defines a first end **112,** a second end **114,** a first edge **116,** a second edge **118,** and four corners **119.** Elongated composite **(102)** includes a length **120** and a width **122.** Length **120** is defined by the distance between first end **112** and second end **114.** Width **122** is defined by the distance between first edge **116** and second edge **118.** Delivery system **104** includes a first support network **120,** a second support network **122,** and a third support network **124** releasably attached to second surface **(108).** Third support network **124** separates first support network **120** from second support network **122** which exposes exposed surfaces **126A** and **126B** of elongated composite **(102).** The exposed surfaces **126A** and **126B** are highly flexible and stretchy portions of the elongated composite.

In some embodiments, wound dressing **100** includes a therapeutic layer **128** having a therapeutic length **130** and a therapeutic width **132.** As shown, the perimeter of the therapeutic layer is removed from, i.e., less than, perimeter **110.** Therapeutic layer **128** may be separated from perimeter **110** by end distances **134A** and **134B,** and by edge distances **136A** and **136B.** A user of wound dressing **100** may cut elongated composite **(102)** along width **112** in order to remove one or more end distances **134A** and **134B** to provide a wound dressing capable of being applied end-long with another wound dressing without interrupting the wound-coverage area of therapeutic layer **128.** Cutting the end distances from the dressing results in a both a modified dressing and an end distance cut portion that have handling tabs on opposite edges of the cut for both pieces which enables handling of these two pieces to further cover and seal the trauma site.

In some embodiments, wound dressing **100** includes one or more end handling tabs **138A** and **138B,** one or more edge handling tabs **140A, 140B, 140C,** and **140D,** or any combination thereof.

**FIG. 1B** illustrates a cross-sectional view of wound dressing **100** including elongated composite **102** and delivery system **104.** Elongated composite **102** includes flexible substrate **106** and therapeutic layer **128** and release liner **144.**

**FIG. 1C** illustrates a cross-sectional view of wound dressing **100** including elongated composite **102,** delivery system **104,** flexible substrate **106,** and therapeutic layer **128.** Wound dressing **100** may further include a perforated layer **142** and a release liner **144.**

**FIG. 2** illustrates a wound dressing **200.** Wound dressing **200** includes all numbered the features of wound dressing **100,** including those numbered accordingly. Some numbers are omitted for clarity. Wound dressing **200** includes a delivery system **204** that includes a first support network **220,** a second support network **222,** a third support network **224,** and a fourth support network **225** releasably attached to the second surface of the flexible substrate (not shown). Third support network **224** and fourth support network **225** separate first support network **220** from second support network **222** which exposes exposed surfaces **226A, 226B,** and **227C** of the second surface. Cross-sectional views (not shown) of wound dressing **200** are similar to those shown in **FIGs. 1A** and **1B****.**

**FIG. 3** illustrates a wound dressing **300.** Wound dressing **300** includes all numbered features of wound dressing **100,** including those numbered accordingly. Wound dressing **300** further illustrates first support network **320** including a first hub **346.** First hub **346** includes phalange(s) **348** extending from first hub **346** to perimeter **310.** As shown, phalange(s) **348** extend to first edge **316** and second edge **318.** Exposed phalange surfaces **349** separate phalanges **348** exposing the second surface of the flexible substrate **306.** Second support network **322** includes a second hub **350.** Second hub **350** includes phalange(s) **352** extending from second hub **350** to perimeter **310.** As shown, phalange(s) **352** extend to first edge **316** and second edge **318** (approaching corners). Third support network **324** includes a third hub **354.** Third hub **354** includes phalange(s) **356** extending from third hub **354** to perimeter **310.** As shown, a portion of the support networks overlap with therapeutic layer **328.** Wound dressing **300** further includes edge handling tabs **340A, 340B, 340C,** and **340D** formed from phalanges **348** and **352.**

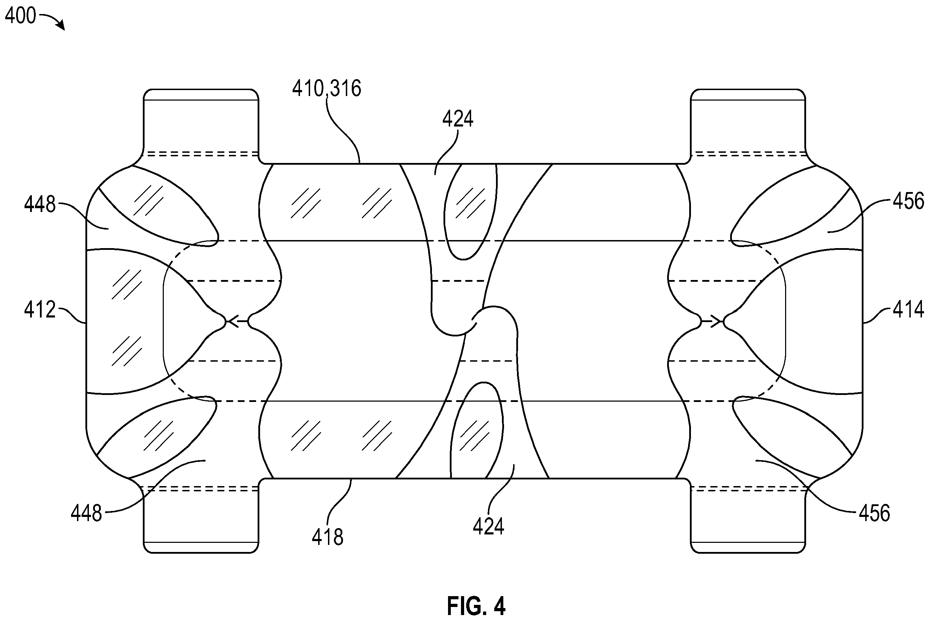

**FIG. 4** illustrates a wound dressing **400.** Wound dressing **400** includes all numbered features of wound dressing **100** and **300,** including those numbered accordingly. Wound dressing **400** differs from that of wound dressing **300,** at least in part, in that the phalange(s) **448** and **456** extend to perimeter **410** at first edge **416,** second edge **418,** first end **412,** and second end **414.** Wound dressing **400** further includes additional third support network **424** phalange(s) **456** as compared to wound dressing **300.**

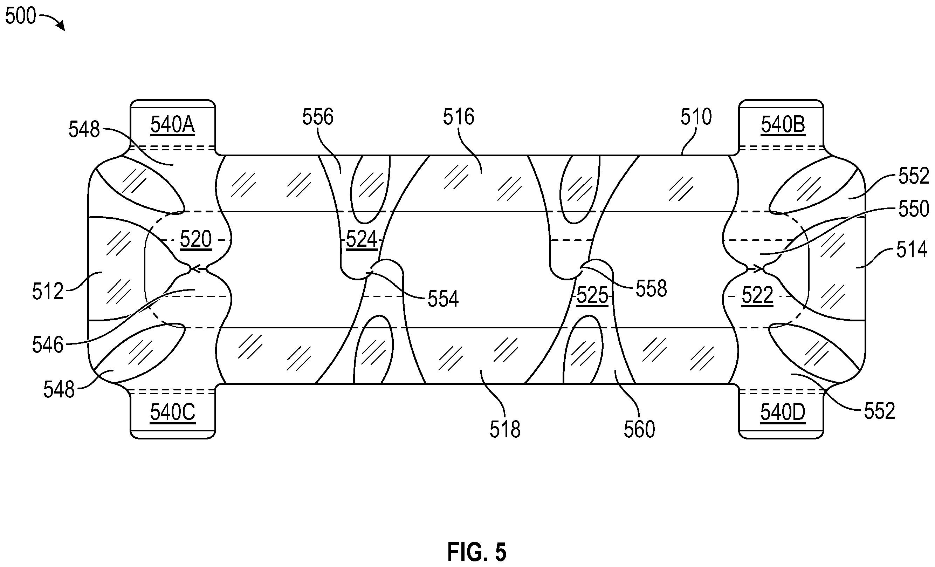

**FIG. 5** illustrates a wound dressing **500.** Wound dressing **500** includes all numbered features of wound dressing **200,** including those numbered accordingly. Wound dressing **500** further illustrates first support network **520** including a first hub **546.** First hub **546** includes phalange(s) **548** extending from first hub **546** to perimeter **510.** As shown, phalange(s) **548** extend to first edge **516,** second edge **518,** first end **512,** and second end **514.** Second support network **522** includes a second hub **550.** Second hub **550** includes phalange(s) **552** extending from second hub **550** to perimeter **510.** As shown, phalange(s) **552** extend to first edge **516,** second edge **518,** first end **512,** and second end **514.** Third support network **524** includes a third hub **554.** Third hub **554** includes phalange(s) **556** extending from third hub **554** to perimeter **510.** Fourth support network **525** includes a fourth hub **558.** Fourth hub **558** includes phalange(s) **560** extending to perimeter **510.** Wound dressing **500** further includes edge handling tabs **540A, 540B, 540C,** and **540D.**

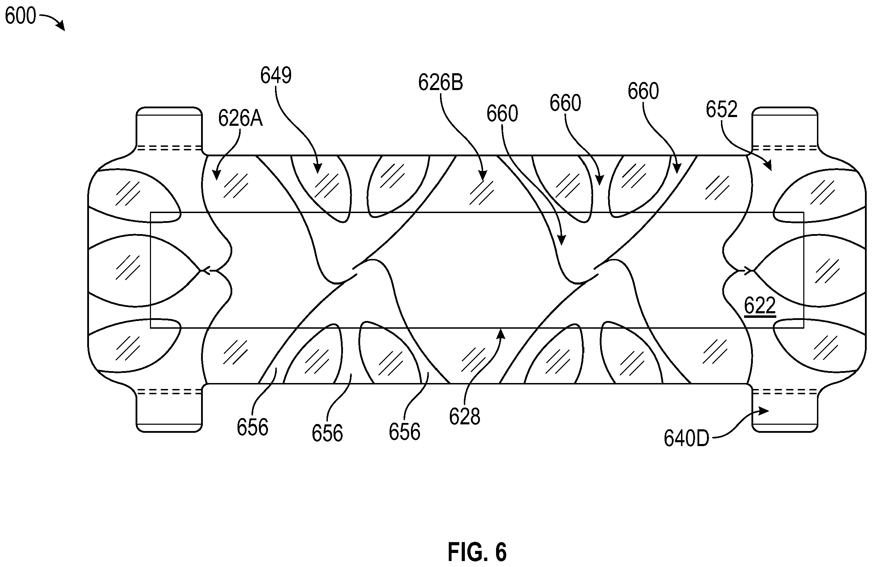

**FIG. 6** illustrates a wound dressing **600.** Wound dressing **600** includes all numbered features of wound dressings **200** and **500,** including those numbered accordingly. Wound dressing **600** differs from wound dressing **500** in that it includes additional phalange(s) **656** and **660.** Also shown here is phalange **652** of second network **622,** handling tab **640D,** exposed surfaces **626A** and **626B, and** exposed phalange surface(s) **649** which separate phalanges exposing the second surface of the flexible substrate.

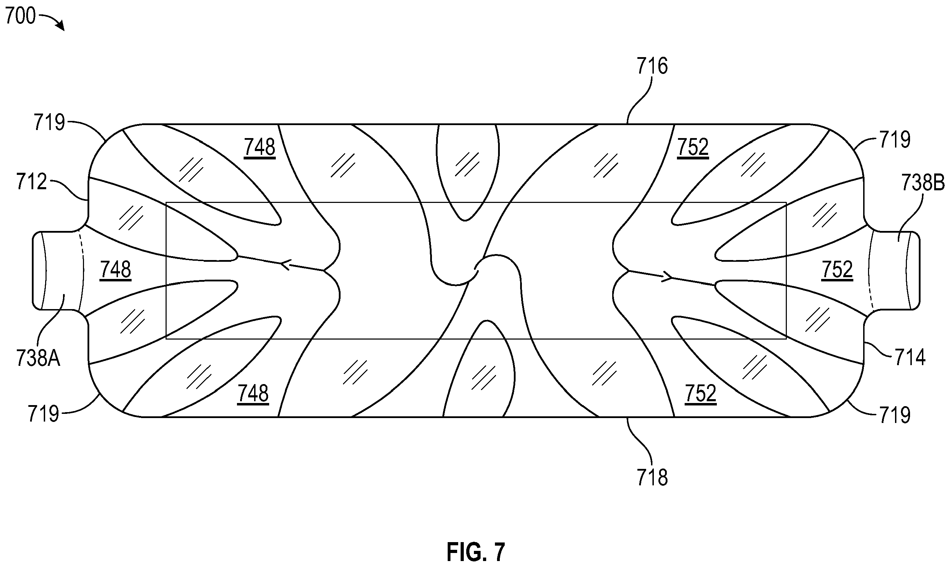

**FIG. 7** illustrates a wound dressing **700.** Wound dressing **700** includes all numbered features of wound dressings **100, 300,** and **400,** including those numbered accordingly. Wound dressing **700** differs from wound dressing **400** in that it includes additional phalange(s) **748** and **752,** wherein phalanges **748** and **752** extend to first end **712,** second end **714,** first edge **716,** second edge **718,** and corner(s) **719.** Wound dressing **700** further differs from wound dressing **400** in that in includes end handling tabs **738A** and **738B** instead of edge handling tabs.

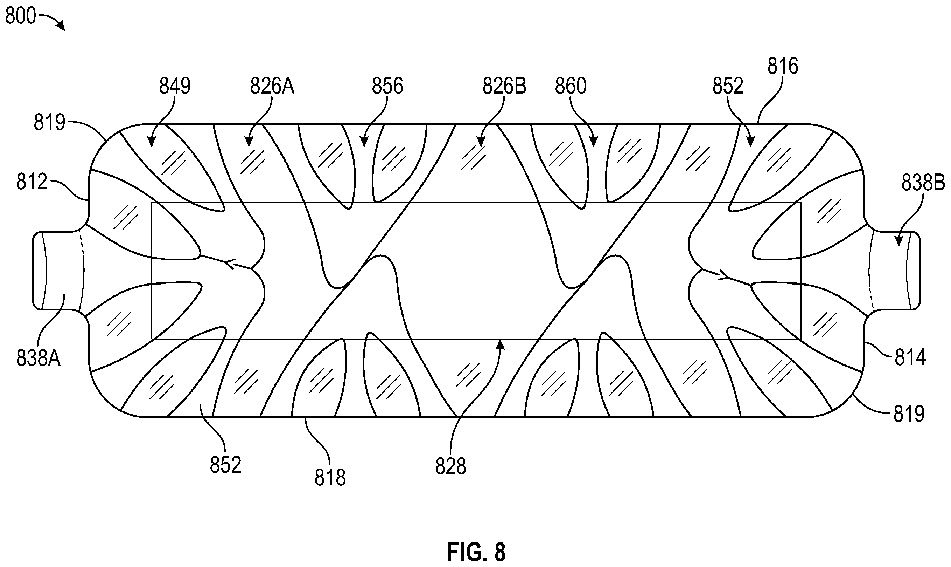

**FIG. 8** illustrates a wound dressing **800.** Wound dressing **800** includes all numbered features of wound dressings **200, 500,** and **600,** including those numbered accordingly. Wound dressing **800** differs from wound dressing **600** in that it includes addition phalanges **848** and **852,** wherein phalanges **848** and **852** extend to first end **812,** second end **814,** first edge **816,** second edge **818,** and corner(s) **819.** Wound dressing **800** further differs from wound dressing **600** in that in includes end handling tabs **838A** and **838B** instead of edge handling tabs.

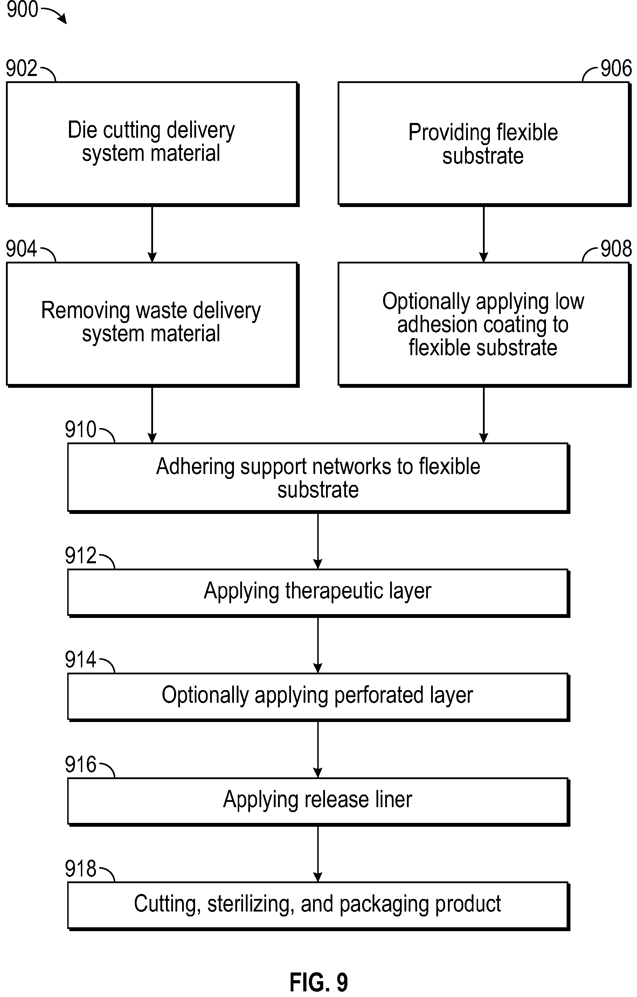

**FIG. 9** outlines a method **900** for the manufacturing of a wound dressing(s) described herein. A delivery system material is die cut **902** according to a predetermine pattern to provide delivery system support networks. Waste delivery system material is removed from the delivery system support networks **904.** A flexible substrate is provided **906.** A low adhesion coating is optionally applied **908** to a second surface of the flexible substrate. The delivery system support networks are further adhered **910** to the flexible substrate. A therapeutic layer is applied **912** to the first surface of the flexible substrate. An optional perforated layer is applied **914** over the therapeutic layer, and a release liner is applied **916** over the perforated layer. The product is cut, sterilized, and packaged **918.**

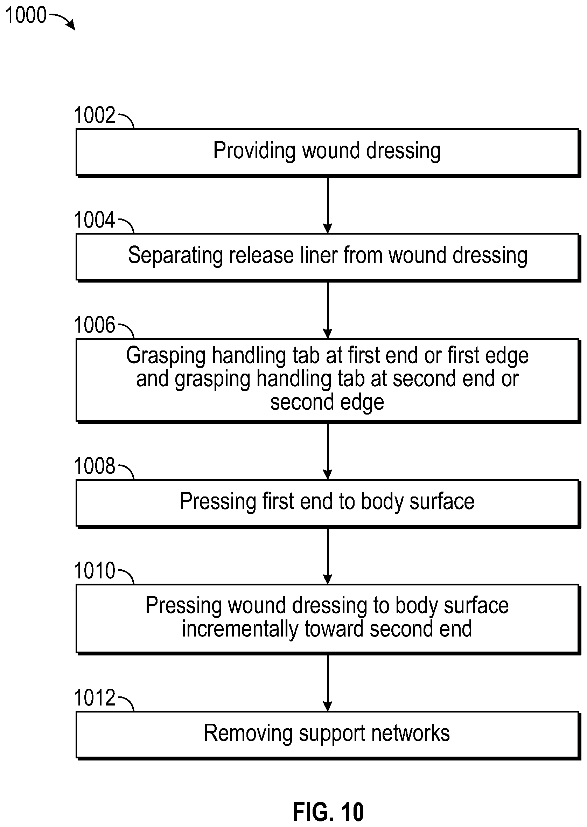

**FIG. 10** outlines a method **1000** for using a wound dressing(s) described herein. The method includes providing the wound dressing **1002.** The release liner is separated from the dressing **1004.** A user(s) grasps a handling tab at the first end or first edge and grasps a handling tab at the second end or second edge **1006.** The first end of the wound dressing is pressed to a body surface and the wound dressing is further pressed to the body surface incrementally toward the second end until the application is complete **1008.** The support network is removed from the dressing **1010.**

### Elongated Composites

In many embodiments, the elongated composite may include a flexible substrate including a first surface and a second surface opposite the first surface; a perimeter including a first end, a second end opposite the first end, a first edge and a second edge opposite the first edge; a length defined by a distance between the first end and the second end; and a width defined by a distance between the first edge and the second edge.

In some embodiments, the elongated composite may include a therapeutic layer. The therapeutic layer may contact the first surface of the flexible substrate.

In some embodiments, the elongated composite may include a perforated layer. The perforated layer may contact one or more of the first surface of the flexible substrate and a therapeutic layer.

In some embodiments, the elongated composite may include a release liner. The release liner may contact one or more of the first surface of the flexible substrate, a therapeutic layer, and a perforated layer.

### Dimensions

In some embodiments, the elongated composite may include a length value of between about 200 mm to about 380 mm. For example, the length may be a value in mm of about 200, 210, 220, 230, 240, 250, 260, 270, 280, 290, 300, 310, 320, 330, 340, 350, 351, 352, 353, 354, 355, 356, 357, 358, 359, or 360, or a value between a range of any of the preceding values. For example, the length may be between about 352 mm and about 353 mm, e.g., 352.5 mm; between about 351 mm and about 353 mm, between about 350 mm to about 360, or the like.

In some embodiments, the elongated composite may include a width of about 85 mm to about 115 mm. For example, the width may be a value in mm of about 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, or 115, or a value between a range of any of the preceding values. For example, the width may be between about 100 mm and 115 mm, between about 102 mm to about 114 mm, or the like.

In some embodiments, the elongated composite may include a length to width ratio of about 2:1 to about 4.5:1. For example, the ratio may be a value of about 2:1, 2.1:1, 2.2:1, 2.3:1, 2.4:1, 2.5:1, 2.6:1, 2.7:1, 2.8:1, 2.9:1, 3:1, 3.1:1, 3.2:1, 3.3:1, 3.4:1, 3.5:1, 3.6:1, 3.7:1, 3.8:1, 3.9:1, 4:1, 4.1:1, 4.2:1, 4.3:1, 4.4:1, or 4:5:1, or a value between a range of any of the preceding values, for example, between about 3:1 to about 4:1, or the like.

In some embodiments, the dimensions of the elongated composite are equal to dimensions of the flexible substrate. The flexible substrate dimensions may be any of the preceding values or within any preceding ranges of values.

### Flexible Substrate

In many embodiments, the flexible substrate may be conformable and resilient. For example, the flexible substrate may conform to nonplanar anatomical surfaces, e.g., knees, elbows, or the like. The flexible substrate may be capable of stretching to accommodate movement of a joint or spine, while providing enough resilience to maintain conformability when the joint is returned to an unflexed position. In some embodiments, the flexible substrate has an ultimate elongation of greater than 200%. In other embodiments, the flexible substrate has an ultimate elongation of greater than 400%. Further, for example, the flexible substrate may allow for guided curvature in order to adequately cover nonlinear wounds with a linear wound dressing.

Suitable materials for flexible substrates include, for example, nonwoven fibrous webs, woven fibrous webs, knits, and films. In many embodiments, flexible substrates of the present disclosure include elastomeric polyurethane, co-polyester, and/or polyether block amide films. These films combine the desirable properties of conformability, high moisture vapor permeability, and transparency desired in certain flexible substrates. Exemplary flexible substrate materials may include thin moisture vapor permeable films made from polyurethane resins such as ESTANE 58237 and ESTANE 58309 (Lubrizol, Brecksville, OH) or ELASTOLLAN 1170A or 1185A (BASF, Ludwigshafen, Germany). In some embodiments, the films may have a thickness between about 15 and about 35 microns. The film thickness may be in microns about 15, 20, 25, 30, or 35, or within a range between any of the preceding values, for example, between about 20 and about 25, or the like.

In some embodiments, the flexible substrate may be made up of a single layer. In other embodiments, the flexible substrate may include more than one layer of one or more materials. The flexible substrate is preferably impermeable to liquids but permeable to moisture vapor. In some embodiments, the flexible substrate is translucent or transparent (e.g., transparent polymeric elastic film). Other layers that may be combined with a thin polyurethane film include, for example, elastic non-wovens made from blends of KRATON G1643 (Kraton Corporation, Houston, Texas) and polypropylene, polyester, or nylon.

In various embodiments, the flexible substrate may be coated with a pressure-sensitive adhesive on the first surface (facing the skin). The pressure-sensitive adhesive may include one or more of an acrylate copolymer, a rubber-based polymer (e.g., tackified natural rubbers, synthetic rubbers, and styrene block copolymers), a polyurethane polymer, and a silicone-based polymer. In some embodiments, an acrylic adhesive may include a copolymer of at least one C4-C12 alkyl (meth)acrylate such as isooctyl acrylate or 2-ethylhexylacrylate and at least one high Tg (e.g. polar) co-monomer such as (meth)acrylamide, N-vinyl pyrrolidone, poly(ethylene oxide)acrylate, or a mixture thereof. In some embodiments, an acrylic adhesive may include at least 90 wt.-% C4-C12 alkyl (meth)acrylate(s). Suitable examples include a 90:10 isooctyl acrylate to acrylic acid copolymer, a 70:15:15 isooctyl acrylate to ethylene oxide acrylate to acrylic acid terpolymer, and/or a 25:69:6 2-ethylhexylacrylate to butyl acrylate to acrylic acid terpolymer. Another example acrylic adhesive may include a 65:15:20 2-ethylhexylacrylate to acrylic acid to copolymer (97:3 isooctyl acrylate to acrylamide copolymer) blended with a nonreactive polyalkylene oxide copolymer, such as the adhesive under the trade designation PLURONIC^{®} (BASF Corporation; Ludwigshafen, Germany).

In some embodiments, the flexible substrate may be coated with a low adhesion coating on the second surface. The low adhesion coating may serve to minimize friction and reduce adhesion of tapes or other securing devices. For example, the low adhesion coating may reduce the number of dressing changes caused by undesired dressing removal when tapes or other securing devices are removed. Further, the low adhesion coating may limit friction between the dressing on linens or other fabrics, thereby offering additional protection from accidental removal. In some aspects, the low adhesion coating may facilitate bonding of the delivery system to the flexible substrate. The low adhesion coating may cover at least a portion of the second surface. Suitable low adhesion coatings can be found, for example, in U.S. Pat. No. 5,531,855,

In some embodiments, the pressure-sensitive adhesives may be silicone-based. Silicone-based adhesives effectively secure dressings to skin and produce little or no skin damage upon removal. Examples of a suitable pressure sensitive silicone gel adhesive are radiation cured silicone oils or gums that include silicate tackifier resins. Generally, the silicone oils or gums may include polydiorganosiloxanes, i.e., materials comprising a polysiloxane backbone. In some embodiments, the nonfunctionalized silicone materials can be a linear material comprising a siloxane backbone with aliphatic and/or aromatic substituents. In some embodiments, the nonfunctionalized polydiorganosiloxane materials may include branched substituents. Suitable silicate tackifying resins include those include MQ silicate tackifying resins, MQD silicate tackifying resins, and MQT silicate tackifying resins. These silicate tackifying resins usually have a number average molecular weight in the range of 100 to 50,000 g/mol, e.g., 500 to 15,000 g/mol. Suitable silicate tackifying resins are commercially available from sources such as Dow Corning (Midland, MI) (e.g., DC2-7066), Momentive Performance Materials (Waterford, NY) (e.g., SR545 and SR1000), and Wacker Chemie AG (Munich, Germany) (e.g., BELSIL TMS-803).

Other examples of silicone adhesives may include products marketed with the trade names: Dow Corning MG 7-9850 (Dow Corning, Midland, MI), Wacker SILPURAN.RTM. 2110 and 2130 (Wacker Chemie AG, Munich, Germany), Bluestar SILBIONE.RTM. RT Gel 4317 and 4320 (Bluestar Silicone USA Corp, East Brunswick, NJ), Nusil MED-6345 and 6350 (Nusil Technology LLC, Carpinteria, CA).

For skin-contact adhesives, it is desirable for the adhesive to transmit moisture vapor at a rate greater to or equal to that of human skin. While such characteristics can be achieved through selection of appropriate adhesives, it is also contemplated that other methods of achieving a high relative rate of moisture vapor transmission may be used, such as perforating the adhesive or pattern-coating the adhesive. Each of the securing or skin-contact adhesives can optionally be applied in a discontinuous manner.

Inclusion of medicaments or antimicrobial agents in the adhesive is also contemplated. Examples of suitable medicaments include chlorhexidine gluconate and silver-based compounds, such as silver oxide, silver chloride, and silver sulfate.

### Therapeutic layer

In various embodiments, the elongated composite may further include a therapeutic layer.

In several embodiments, the therapeutic layer extends between and underneath at least a portion of the each of the hubs of the various support networks to act as a bridge or stiffening agent between the support networks. This added stiffening reduces the chances that the flexible substrate will fold onto itself during application of the dressing to a wound site.

In some embodiments, the therapeutic layer may have the same general shape as the elongated composite and/or flexible substrate.

In some embodiments, the therapeutic layer may be removed from the perimeter of the elongated composite and/or flexible substrate. In some embodiments, the therapeutic layer may have the same general shape as the elongated composite and/or flexible substrate. In many embodiments, the therapeutic layer may have a perimeter value less than the perimeter value of the elongated composite, resulting in an island-type dressing. The perimeter of the therapeutic layer may be less than 90, 85, 80, 75, 70, 65, 60, 55, or 50 percent of the elongated composite perimeter, or a value between a range of any of the preceding values. In some embodiments, the perimeter of the therapeutic layer is less than 50 percent of the elongated composite perimeter.

In many embodiments, the therapeutic layer is removed from the first end and the second end by an end distance. This may allow a user may cut away one or more end distance(s) in order to apply consecutive wound dressings to cover exceptionally long wounds or traumas with uninterrupted therapeutic layers. Each end distance may independently be about 15 to about 35 mm. For example, each end distance may independently be in mm of about 15, 20, 25, 30, or 35, or within range of any of the preceding values, for example, between about 20 and about 30, between about 20 and 25, or the like.

In many embodiments, the therapeutic layer is removed from the first edge and the second edge by an edge distance. Each edge distance may independently be about 15 to about 35 mm. For example, each edge distance may independently be in mm of about 15, 20, 25, 30, or 35, or within range of any of the preceding values, for example, between about 20 and about 30, between about 20 and 25, or the like.

In several embodiments, the therapeutic layer may include one or more areas wherein the material is interrupted, e.g., by partial or full cuts, perforations, fenestrations, gaps, thinner material, or the like. Such areas may provide additional flexibility and allow for the wound dressing to better maneuver along nonlinear surfaces upon application of the wound dressing. In some embodiments, the interruptions may be approximately parallel with the ends of the therapeutic layer. In other embodiments, the interruptions may be at an angle of less than 45 degrees relative to the orientation of the end of the therapeutic layer. In many embodiments, the interruptions do not extend from one edge to the other edge of the therapeutic layer.

In various embodiments, the therapeutic layer may include an absorbent pad. The absorbent pad may include one or more layers, and each layer may include one or more absorbent materials. In some embodiments, the absorbent pad is flexible.

In some embodiments, the absorbent pad may be translucent or transparent, thus allowing for visual inspection of the wound without removal of the wound dressing.

In many embodiments, the absorbent pad may include synthetic or natural materials, including woven or nonwoven materials (e.g., woven or nonwoven cotton or rayon), hydrocolloids (e.g., pectin, gelatin, carboxymethylcellulose (CMC), cross-linked carboxymethylcellulose (X-link CMC), cross-linked polyacrylic acid (PAA)), polymer gels (e.g., hydrogels), foams, collagens, hydrofibers, alginates, and combinations thereof. In some embodiments, the absorbent pad may include a polymeric fabric, a polymeric foam, and combinations thereof. For example, the polymeric fabric may be a nonwoven and the polymeric foam may be the foam used in the TEGADERM foam adhesive dressing available from 3M Company, St. Paul, Minn. In certain embodiments, the polymeric foam is a polyurethane foam.

In certain embodiments, the absorbent pad may include an absorbent hydrogel. The absorbent hydrogel may include a swellable, crosslinked poly(N-vinyl lactam), such as crosslinked polyvinylpyrrolidone, a swelling agent such as polyglycerol-3, and an optional modifying polymer such as hydroxypropyl guar present in an amount sufficient to form a cohesive, absorbent hydrogel composition. The swellable poly(N-vinyl lactam) may be present in an amount from about 10 to about 50 wt% of the composition. When the poly(N-vinyl lactam) is poly(N-vinyl pyrrolidone), poly(N-vinyl pyrrolidone) may be present in an amount from about 15 to about 45 wt% of the composition. In some embodiments, the poly (N-vinyl pyrrolidone) may be present in an amount from about 18 to about 35 wt% of the composition. In some embodiments, the amount of swelling agent may be present in an amount from about 50 to about 90 wt % of the composition. In some embodiments, the optional modifying polymer may be present in an amount of about less than 5 wt % of the composition.

In many embodiments, the absorbent hydrogel may include less than 25 wt% water with respect to the composition.

In many embodiments, the absorbent pad may include one or more active agents. For example, the absorbent pad may include growth factors (e.g., TGF, FGF, PDGF, EGF, or the like), antibacterial agents (e.g., penicillin, neomycin sulfate, sulfonamides, sulfadiazine, silver sulfadiazine, trimethoprim, povidone iodine, iodine, silver, silver chloride, chlorhexidine (e.g., chlorhexidine gluconate), or the like), antifungal agents (e.g., griseofulvin, chlormidazole hydrochloride, clotrimazole, ketoconazole, miconazole, miconazole nitrate, nistatin, tolnaftate, or the like), disinfectants and antiseptics (e.g., benzalkonium chloride, cetalkonium chloride, chlorhexidine gluconate, ethanol, iodine, methylbenzethonium, povidone iodine, isopropanol, silver, silver oxide, silver salts such as silver lactate and silver chloride, triclosan, or the like), local anesthetics (e.g., tetracaine, benzocaine, prilocaine, procaine, or the like), debriding agents, anti-inflammatory agents (e.g., indomethacin, ketoprofen, dichlofenac, ibuprofen, or the like), astringents, enzymes, nutrients (e.g., vitamins, minerals, oxygen, or the like), drugs for cataplasms (e.g., menthol, camphor, peppermint, capsicum extract, capsaicin, or the like), and odor absorbing agents (e.g., zeolites, silicates, chitosans, cyclodextrins, or the like).

In many embodiments, the absorbent pad may include chlorhexidine gluconate.

### Perforated layer

In some embodiments, the elongated composite may include a perforated layer.

In some embodiment, the perforated layer may be in at least partial contact with the first surface of the flexible substrate. The perforated layer may be in at least partial contact with the therapeutic layer.

In some embodiments, the perforated layer may be liquid permeable. In some embodiments, the perforated layer may be applied discontinuously (e.g., pattern coated, perforated or slit) such that liquid or wound exudate can pass through the perforate layer and reach the therapeutic layer. In some embodiments, the perforated layer is at least 5% open, i.e., at least 5% perforated. In other embodiments, the perforated layer is at least 15% open.

In some embodiments the perforated layer is coextensive with the elongated composite. In some embodiments, the perforated layer is coextensive with the therapeutic layer.

The perforated layer is preferably soft, flexible, conformable, non-irritating and non-sensitizing. The perforated layer is preferably transparent to allow for visual inspection of the wound site.

In some embodiments, the perforated layer may include an adhesive on one or more surfaces of a perforated layer. The adhesive may facilitate bonding of the perforate layer to one or more of the flexible substrate, therapeutic layer, and/or wound site. The adhesive may include any adhesive described herein. The perforated layer may be a porous adhesive layer. The adhesive may include any adhesive described herein. In some embodiments, the perforated layer may prevent adhesion to the wound.

In some embodiments, the perforated layer may include a moisture vapor permeable film, perforated film, porous film, woven, non-woven or knit webs, or scrims. The perforated layer may include polyurethane, polyethylene, polypropylene, polyamide or polyester materials. For example, the perforated layer may include polyurethane films such as those available under the trade designation ESTANE (Lubrizol in Brecksville, Ohio); elastomeric polyesters such as those available under the trade designation HYTREL (E.I. duPont deNemours & Co., Wilmington, Del.); and/or polyether block amides such as those available under the trade designation PEBAX (Elf Altochem North America, Philadelphia, Pa). Other useful films include those made from polyurethane resins under the trade designation ELASTOLLAN (BASF, Ludwigshafen, Germany).

In some embodiments, the perforated layer may be between about 15 microns and about 200 microns thick. In some embodiments, the perforated layer may be between about 15 and about 35 microns thick. In some embodiments, the perforated layer may have an ultimate elongation of at least 200%. In other embodiments, the perforated layer may have an ultimate elongation of at least 400%.

### Release Liner

In several embodiments, the elongated composite may further include a release liner. The release liner may be releasably attached to one or more of the flexible substrate, therapeutic layer, and perforated layer. The release liner is removed to expose a wound contact surface prior to application of the wound dressing.

Release liners may include kraft papers, polyolefin, polyethylene, polypropylene, polyester or composites of any of these materials. In some embodiments, the release liners may be coated with one or more release agents, e.g., fluorochemicals or silicones. The release liner may be any commercially available release liner such POLYSLIK^{™} silicone release papers available from Loparex (Cary, North Carolina); Silicone 1750 coated films from Infiana (Forchheim, Germany), and 3M Scotchpak^{™} 9741 Release liner from 3M Company (St. Paul, Minnesota).

### Delivery Systems

In various embodiments, the wound dressings of the present disclosure include a delivery system. The delivery system may include a plurality of support networks capable of assisting a user to deliver the wound dressing to a subject. The support networks allow for coverage of long wounds, especially nonplanar wounds that require dressing conformability and resiliency. The delivery system provides stability of the wound dressing for proper application yet enables high conformability and resiliency of the dressing. Without delivery systems, such wound dressings would fold onto themselves, wrinkle, and/or otherwise fail to adequately cover the wound.

### Support Networks

In various embodiments, the delivery system includes a first support network, a second support network, and a third support network.

In some embodiments, the delivery system may further include a fourth support network.

The number of support networks for adequate stability depends upon, for example, the dimensions of the wound dressing and the degree of flexibility in the elongated composite. Longer wound dressings tend to require more support networks.

In some embodiments, the delivery system may include more than four support networks.

In several embodiments, the support networks may be separated from each other by exposing the flexible substrate surface. The distance between any two adjacent support networks, e.g., first and third, third and second, third and fourth, fourth and second, may be between about 10 mm and about 100 mm. In some embodiments the distance between any two adjacent support networks may be in mm about 10, 15, 20, 25, 30, 35, 40, 45, 50, 55, 60, 65, 70, 75, 80, 85, 90, 95 or 100, or within a range between any of the preceding values, e.g., between about 40 mm and about 60 mm, between about 20 mm and about 50 mm, or the like. In some embodiments, the distance between any adjacent support network is no greater than about 150 mm. In many embodiments, the distances between adjacent support networks may be the same or different. Any combination of distances between adjacent support networks cannot exceed the length of the elongated composite described herein.

In many embodiments, each support networks may include a hub, e.g., a first, second, third, and fourth hub, respectively. Each hub may be medially nested along the width of the elongated composite.

In some embodiments, one or more hubs are perforated, have cuts, or lines of weakness to allow for removal of a portion of the support network independent of the entire support network.

In many embodiments, each hub may include at least one phalange extending from the hub to the perimeter of the elongated composite and/or flexible substrate. The at least one phalange may extend to the first end, the second end, the first edge, the second edge, or any corner thereof. Exceptional stability is noted with phalanges extending to or near corners.

In several embodiments, a hub may include a plurality of phalanges. The number of phalanges may be the same or different for each respective hub. In some embodiments, a hub may include 1 to 8 phalanges, e.g., 1, 2, 3, 4, 5, 6, 7, or 8, or a value or range between any of the preceding values, e.g., between 2 and 6, or the like. For example, a first hub may include 4 phalanges and a third hub may include 6 phalanges, or the like.

In several embodiments, one or more phalanges of the first and/or second support network may extend to the first and second ends, respectively.

In several embodiments, one or more phalanges of the first and/or second support networks may extend to the first and second edges, respectively.

In several embodiments, one or more phalanges of the first and second support networks may extend to a corner.

In several embodiments, the first and/or second support networks may include one or more phalanges extending to one or more of the respective ends, edges, and corners.

In many embodiments, one or more of the first support network and the second support network may include at least one phalange extending beyond the perimeter to form a handling tab. The at least one phalange may extend past the first end, second, end, first edge, second edge, corners. In some embodiments, the first end and second ends may include at least one handling tab each. In some embodiments, the first edge and second edge may include at least one handling tab. In some embodiments, the first edge and second edge may include two handling tabs each. In some embodiments, the first end and second end may each include a handling tab, and the first edge and second edge may each include at least one handling tab.

In some embodiments, a straight line passing from one or more handling tabs to the opposite side traverses the therapeutic layer. For example, first support network handling tabs at the first edge and/or second edge may be positioned such that at least one line passing through any part of the tab(s) traverses the therapeutic layer.

In several embodiments, the third and/or fourth support networks may include one or more phalanges extending to the first and second edges.

In many embodiments, the plurality of phalanges of a give support network may be separated by exposed surface of the flexible substrate.

In some embodiments, a phalange may have a nonuniform width extending from a hub to the perimeter. For example, a phalange may be narrower proximal to the hub and wider distal to the hub. In other embodiments, a phalange may have a uniform width extending from a hub to the perimeter. In some embodiments, phalanges of a delivery system may include only uniform widths, only nonuniform widths, or both uniform and nonuniform widths.

In some embodiments, any given support network may be symmetrical, e.g., with respect to one or more of hub shape and phalange shape and count. In other embodiments, any given support network may be unsymmetrical.

In some embodiments, some support networks may be symmetrical with other support networks, e.g., first and second support networks, or third and fourth support networks, may be symmetrical with each other. In other embodiments, some support networks may be unsymmetrical with other support networks.

In many embodiments, the support networks are made from a material more rigid than that of the flexible substrate to prevent the flexible substrate from wrinkling and/or folding during application.

In some embodiments, the support networks may include one or more materials selected from polyethylene/vinyl acetate copolymer, polyvinylacetate-coated paper, polyester film, and polyethylene film. The support networks may further include materials such as nonwovens, polymer films, and papers. One example of a support network material includes a polyethylene/vinyl acetate copolymer coated super calendared Kraft paper available from Loparex (Cary, N.C.). Other examples of suitable support network materials include polyethylene/vinyl acetate copolymer coated polyester film, polyvinylacetate coated polyester film, or polyethylene film. In some embodiments, the support networks may further include non-wovens, polymer films, or papers attached to the aforementioned polymer films at distinct locations. One example of a suitable support network material includes the polyester coated film used in the manufacture of the 90024 3M^{™} TEGADERM^{™} Hydrocolloid Thin Dressing available from 3M.

In some embodiments, each support network may independently have a thickness between about 15 and about 125 microns.

In some embodiments, one or more support network may independently include a coated polyester film having a thickness of between about 15 and about 55 microns.

In some embodiments, one or more support network may independently include a polyvinylacetate coated polyester film having thickness of between 20 and 125 microns thick.

In some embodiments, a support network may have a uniform thickness. In other embodiments, a support network may have a non-uniform thickness.

In some embodiments, the support networks may be heat-sealable to the flexible substrate with or without a low adhesion coating described herein.

In some embodiments, a bond block material may be located under a portion of each respective hub to prevent bonding of the hub to the second surface of the flexible substrate. This lack of bond at a portion of the hub facilitates removal of the support network after application of the dressing to the trauma site. The bond block material may be paper that is adhered to the underside of a portion of the hub.

### Methods of Manufacture

In various embodiments, a method of manufacturing a wound dressing is described. The method may include die cutting a delivery system material to a predetermined pattern to provide a plurality of delivery system support networks. The delivery system material may be any delivery system material described herein. In some embodiments, the die cutting may be afforded using rotary die cutting equipment.

In many embodiments, the method may include removing waste material from the die cutting. The waste removal may involve, for example, the use of a vacuum, air pressure, gravity, and/or small diameter nip rolls.

In many embodiments, the method may include providing a flexible substrate described herein. A low adhesion coating may optionally be applied to a surface of the flexible substrate.

In many embodiments, the method may then include adhering the delivery system support networks to the flexible substrate. For example, the adhering may include heat sealing.

In some embodiments, the method may further include applying a therapeutic layer to the flexible substrate. The therapeutic layer may be any therapeutic layer described herein.

In some embodiments, the method may optionally include applying a perforated layer to one or more of the flexible substrate and therapeutic layer. The perforated layer may be any perforated layer described herein.

In some embodiments, the method may further optionally include applying a release liner to one or more of the flexible substrate, therapeutic layer, and perforated layer. The release liner may be any release liner described herein.

In many embodiments, the resulting wound dressing material may further be cut, e.g., with a rotary die sheeting station, and the individual dressings may be fed into packaging station. Sterilization may occur before or after one or more packaging steps.

In many embodiments, each of the flexible substrate, perforated layer, and release liner may include an adhesive, or an adhesive may be coated thereupon prior to applications.

### Methods of Use

In various embodiments, a method for covering a wound is described. The method may include providing a wound dressing described herein. The method may include separating the release liner from the wound dressing. The method may include grasping a handling tab at the first end or first edge and further grasping a handling tab at the second end or second edge. In some embodiments, the grasping may be done by an individual. In other embodiments, the grasping may be done by more than one person. The method may include pressing the first end to a body surface, e.g., skin surface. The wound dressing may be further pressed to the body surface incrementally toward the second end. In some embodiments, the support networks may be removed after application of the wound dressing. In other embodiments, the support networks may be removed after portions of the dressing are applied.

In some embodiments, removal of the support networks may begin with separating the hubs from the elongated composite.

In some embodiments, the body surface may include broken or disrupted skin, e.g., incisions, burns, scrapes, abrasions, infections, rashes, punctures, cuts, skin growths, or the like. The body surface may be nonplanar. The body surface may include a joint. The broken or disrupted skin surface may be linear or nonlinear. In some embodiments, the broken or disrupted skin surface may have a length of between about 125 mm to about 300 mm.

In some embodiments, the incrementally pressing of the wound dressing toward the second end may allow a user to maneuver the wound dressing along non-linear injuries.

In some embodiments, the method may include pressing a portion of the wound dressing to the body surface and removing the support network which supported that portion prior to pressing a further portion of the wound dressing to the body surface.

During application of the wound dressing, the third and/or fourth support networks facilitate maneuvering of dressing to cover a non-linear wound even over a highly contoured body part. For example, the dressing can be applied to the skin and injury between the first and third support networks. Because of the added rigidity of the dressing at the third support network and the highly flexible area of the exposed surfaces between support networks, the dressing can then be maneuvered or angled in a different direction between the third and second (or fourth) support network because of the highly flexible and stretchy nature of the exposed surfaces area between network support networks. This combination of alternating rigidity and flexibility of a dressing may allow one to better cover or treat a non-linear wound or injury.

In some embodiments, the method may further include cutting two or more elongated composites at one or more end distance and applying an additional wound dressing to the skin surface of the subject such that the therapeutic layers of each wound dressing align for uninterrupted coverage of the broken or disrupted skin surface.

### Definitions

As used herein, the term "about" is defined as ± 10% of a stated value. For example, "about 20" may mean 18 to 22.

As used herein, the term "conformable" describes an object with an ability to adopt the same shape as of another object.

As used herein, the term "hub" is defined as a point of origin or center.

As used herein, the term "nonlinear" is defined as not being straight. With respect to the present disclosure, a nonlinear wound is one that would extend beyond a therapeutic layer when a wound dressing is straightly applied.

As used herein, the term "nonplanar" is defined as not being confined within a single plane. No body part is likely to adhere to the strictest interpretation of such definition. Nonplanar is meant to describe body surfaces that bend out of plane and/or that have a curvature within the dimensions of a given wound dressing.

As used herein, the term "phalange" is defined as an extension from a point of origin.

As used herein, the term "removed" as used in the phrase "removed from the perimeter" means an object, e.g., a therapeutic layer, having dimensions less than said perimeter.

As used herein, the term "resilient" describes an object with an ability to recoil or spring back into shape after bending, stretching, or being compressed.

As used herein, the term "ultimate elongation" means the percentage increase in length that occurs before the object breaks under tension.

As used herein, the term "support network^{™} is defined as an object providing conformational stability to another object. Without support networks, the wound dressings of the present disclosure would collapse upon attempting to apply the wound dressing to a subject.

As used herein, the phrase "a straight line passing from one or more handling tabs to the opposite edge, traverses the therapeutic layer" means that one or more handling tabs is/are positioned such that if one or more end distances were removed from the wound dressing, at least a portion of the handling tabs would remain with the wound dressing since at no point is a user cutting into the therapeutic layer.

## Claims

1. A wound dressing comprising an elongated composite and a delivery system,
the elongated composite comprising:
a flexible substrate comprising a first surface and a second surface opposite the first surface,
a perimeter comprising a first end, a second end opposite the first end, a first edge and a second edge opposite the first edge,
a length defined by a distance between the first end and the second end, and
a width defined by a distance between the first edge and the second edge; and
the delivery system comprising:
a first support network releasably attached to the second surface,
the first support network comprising:
a first hub medially nested along the width, and
at least one phalange extending from the first hub to the perimeter;
a second support network releasably attached to the second surface,
the second support network comprising:
a second hub medially nested along the width, and
at least one phalange extending from the second hub to the perimeter; and
a third support network releasably attached to the second surface,
the third support network comprising:
a third hub medially nested along the width, and
at least one phalange extending from the third hub to the perimeter,
wherein the third support network separates the first support network from the second support network;
wherein each of the first support network, second support network, and third support network are disconnected and separated from adjacent support networks to expose the second surface of the elongated composite.

2. The wound dressing of any one of the preceding claims, further comprising a therapeutic layer adjacent the first surface and removed from the perimeter and extends under at a least a portion of each hub of the support networks.

3. The wound dressing of any one of the preceding claims, one or more of the first support network and the support second support network comprising at least one phalange extending beyond the perimeter to form a handling tab at the first edge and/or second edge.

4. The wound dressing of claim 3, wherein a straight line passing from one or more handling tabs to the opposite edge traverses the therapeutic layer.

5. The wound dressing of any one of the preceding claims, wherein one or more of the first hub, second hub, and third hub are perforated.

6. The wound dressing of any one of the preceding claims, wherein one or more of the first support network and the support second support network comprises two to six phalanges.

7. The wound dressing of any one of the preceding claims, one or more of the first support network and the second support network comprising at least one phalange extending beyond the perimeter to form a handling tab at the first end and/or the second end.

8. The wound dressing of any one of the preceding claims, wherein the third support network comprises two to four phalanges.

9. The wound dressing of any one of the preceding claims, further comprising a fourth support network, the fourth support network comprising: a fourth hub, and one or more phalange extending from the fourth hub to the perimeter, wherein the fourth support network separates the first support network or the second support network from the third support network.

10. The wound dressing of any one of the preceding claims, wherein the ratio of length to width is between 2:1 to 4.5:1.

11. The wound dressing of any one of the preceding claims, wherein any exposed surface between networks is about 10 mm to about 100 mm.

12. The wound dressing of any one of the preceding claims, wherein any exposed second surface is no greater than 150 mm along the length of the elongated composite.

13. The wound dressing of any one of claims 2-12 wherein the therapeutic layer comprises an absorbent hydrogel.

14. The wound dressing of any one of claims 2-13, wherein the therapeutic layer comprises chlorhexidine gluconate.

## Patentansprüche

1. Ein Wundverband, aufweisend einen länglichen Verbundwerkstoff und ein Abgabesystem, wobei der längliche Verbundwerkstoff aufweist:
ein flexibles Substrat, aufweisend eine erste Oberfläche und eine der ersten Oberfläche gegenüberliegende zweite Oberfläche,
einen Umfang, aufweisend ein erstes Ende, ein dem ersten Ende gegenüberliegendes zweites Ende, einen ersten Rand und einen dem ersten Rand gegenüberliegenden zweiten Rand,
eine Länge, die durch einen Abstand zwischen dem ersten Ende und dem zweiten Ende definiert ist, und
eine Breite, die durch einen Abstand zwischen dem ersten Rand und dem zweiten Rand definiert ist; und
wobei das Abgabesystem aufweist:
ein erstes Trägernetz, das an der zweiten Oberfläche lösbar befestigt ist,
wobei das erste Trägernetz aufweist:
einen ersten Ansatz, der entlang der Breite medial verschachtelt ist, und
mindestens eine Strebe, die sich von dem ersten Ansatz zu dem Umfang erstreckt;
ein zweites Trägernetz, das an der zweiten Oberfläche lösbar befestigt ist,
wobei das zweite Trägernetz aufweist:
einen zweiten Ansatz, der entlang der Breite medial verschachtelt ist, und
mindestens eine Strebe, die sich von dem zweiten Ansatz zu dem Umfang erstreckt; und
ein drittes Trägernetz, das an der zweiten Oberfläche lösbar befestigt ist,
wobei das dritte Trägernetz aufweist:
einen dritten Ansatz, der entlang der Breite medial verschachtelt ist, und
mindestens eine Strebe, die sich von dem dritten Ansatz zu dem Umfang erstreckt, wobei das dritte Trägernetz das erste Trägernetz von dem zweiten Trägernetz trennt;
wobei das erste Trägernetz, das zweite Trägernetz und das dritte Trägernetz jeweils von angrenzenden Trägernetzen unterbrochen und getrennt sind, um die zweite Oberfläche des länglichen Verbundwerkstoffs freizulegen.

2. Der Wundverband nach einem der vorstehenden Ansprüche, ferner aufweisend eine therapeutische Schicht, die an die erste Oberfläche angrenzt und von dem Umfang entfernt ist und sich unter mindestens einem Abschnitt jedes Ansatzes der Trägernetze erstreckt.

3. Der Wundverband nach einem der vorstehenden Ansprüche, eines oder mehrere des ersten Trägernetzes und des zweiten Trägernetzes aufweisend mindestens eine Strebe, die sich über den Umfang hinaus erstreckt, um an dem ersten Rand und/oder dem zweiten Rand eine Handhabungslasche auszubilden.

4. Der Wundverband nach Anspruch 3, wobei eine gerade Linie, die von einer oder mehreren Handhabungslaschen zu dem gegenüberliegenden Rand verläuft, die therapeutische Schicht durchquert.

5. Der Wundverband nach einem der vorstehenden Ansprüche, wobei eine oder mehrere des ersten Ansatzes, ders zweiten Ansatzes und des dritten Ansatzes perforiert sind.

6. Der Wundverband nach einem der vorstehenden Ansprüche, wobei eines oder mehrere des ersten Trägernetzes und des zweiten Trägernetzes zwei bis sechs Streben aufweisen.

7. Der Wundverband nach einem der vorstehenden Ansprüche, wobei eines oder mehrere des ersten Trägernetzes und des zweiten Trägernetzes mindestens eine Strebe aufweist/aufweisen, die sich über den Umfang hinaus erstreckt, um an dem ersten Ende und/oder an dem zweiten Ende eine Handhabungslasche auszubilden.

8. Der Wundverband nach einem der vorstehenden Ansprüche, wobei das dritte Trägernetz zwei bis vier Streben aufweist.

9. Der Wundverband nach einem der vorstehenden Ansprüche, ferner aufweisend ein viertes Trägernetz, wobei das vierte Trägernetz aufweist: einen vierten Ansatz und ein oder mehrere Streben, die sich von dem vierten Ansatz zu dem Umfang erstrecken, wobei das vierte Trägernetz das erste Trägernetz oder das zweite Trägernetz von dem dritten Trägernetz trennt.

10. Der Wundverband nach einem der vorstehenden Ansprüche, wobei das Verhältnis von Länge zu Breite zwischen 2:1 und 4,5:1 liegt.

11. Der Wundverband nach einem der vorstehenden Ansprüche, wobei eine beliebige freiliegende Oberfläche zwischen Netzen etwa 10 mm bis etwa 100 mm beträgt.

12. Der Wundverband nach einem der vorstehenden Ansprüche, wobei eine beliebige freiliegende zweite Oberfläche entlang der Länge des länglichen Verbundwerkstoffs nicht größer als 150 mm ist.

13. Der Wundverband nach einem der Ansprüche 2 bis 12, wobei die therapeutische Schicht ein absorbierendes Hydrogel aufweist.

14. Der Wundverband nach einem der Ansprüche 2 bis 13, wobei die therapeutische Schicht Chlorhexidingluconat aufweist.

## Revendications

1. Pansement comprenant un composite allongé et un système d'administration,
le composite allongé comprenant :
un substrat souple comprenant une première surface et une seconde surface opposée à la première surface,
un périmètre comprenant une première extrémité, une seconde extrémité opposée à la première extrémité, un premier bord et un second bord opposé au premier bord,
une longueur définie par une distance entre la première et la seconde extrémité, et
une largeur définie par une distance entre le premier et le second bord ; et
le système d'administration comprenant :
un premier réseau de soutien fixé de manière amovible à la seconde surface,
le premier réseau de soutien comprenant :
un premier moyeu emboîté médialement le long de la largeur, et
au moins une phalange s'étendant du premier moyeu au périmètre ;
un second réseau de soutien fixé de manière amovible à la seconde surface,
le deuxième réseau de soutien comprenant :
un second moyeu emboîté médialement le long de la largeur, et
au moins une phalange s'étendant du second moyeu au périmètre ; et
un troisième réseau de soutien fixé de manière amovible à la seconde surface,
le troisième réseau de soutien comprenant :
un troisième moyeu emboîté médialement le long de la largeur, et
au moins une phalange s'étendant du troisième moyeu au périmètre, dans lequel le troisième réseau de soutien sépare le premier réseau de soutien du deuxième réseau de soutien ;
dans lequel chacun du premier réseau de soutien, du deuxième réseau de soutien et du troisième réseau de soutien sont déconnectés et séparés des réseaux de soutien adjacents afin d'exposer la seconde surface du composite allongé.

2. Pansement selon l'une quelconque des revendications précédentes, comprenant en outre une couche thérapeutique adjacente à la première surface et retirée du périmètre, qui s'étend sous au moins une partie de chaque moyeu des réseaux de soutien.

3. Pansement selon l'une quelconque des revendications précédentes, un ou plusieurs du premier réseau de soutien et du second réseau de soutien comprenant au moins une phalange s'étendant au-delà du périmètre pour former une languette de manipulation au niveau du premier bord et/ou du second bord.

4. Pansement selon la revendication 3, dans lequel la couche thérapeutique est traversée par une ligne droite allant d'une ou plusieurs languettes de manipulation au bord opposé.

5. Pansement selon l'une quelconque des revendications précédentes, dans lequel un ou plusieurs des premiers moyeux, seconds moyeux et troisièmes moyeux sont perforés.

6. Pansement selon l'une quelconque des revendications précédentes, dans lequel un ou plusieurs des premiers réseaux de soutien et des seconds réseaux de soutien comprennent de deux à six phalanges.

7. Pansement selon l'une quelconque des revendications précédentes, un ou plusieurs du premier réseau de soutien et du second réseau de soutien comprenant au moins une phalange s'étendant au-delà du périmètre pour former une languette de manipulation à la première extrémité et/ou à la seconde extrémité.

8. Pansement selon l'une quelconque des revendications précédentes, dans lequel le troisième réseau de soutien comprend deux à quatre phalanges.

9. Pansement selon l'une quelconque des revendications précédentes, comprenant en outre un quatrième réseau de soutien, le quatrième réseau de soutien comprenant : un quatrième moyeu, et une ou plusieurs phalanges s'étendant du quatrième moyeu au périmètre, dans lequel le quatrième réseau de soutien sépare le premier réseau de soutien ou le deuxième réseau de soutien du troisième réseau de soutien.

10. Pansement selon l'une quelconque des revendications précédentes, dans lequel le rapport entre la longueur et la largeur est compris entre 2:1 et 4,5:1.

11. Pansement selon l'une quelconque des revendications précédentes, dans lequel toute surface exposée entre les réseaux est d'environ 10 mm à environ 100 mm.

12. Pansement selon l'une quelconque des revendications précédentes, dans lequel toute seconde surface exposée ne dépasse pas 150 mm sur la longueur du composite allongé.

13. Pansement selon l'une quelconque des revendications 2-12, dans lequel la couche thérapeutique comprend un hydrogel absorbant.

14. Pansement selon l'une quelconque des revendications 2-13, dans lequel la couche thérapeutique comprend un gluconate de chlorhexidine.
